Europäisches Patentamt

European Patent Office

Office européen des brevets

(11) Veröffentlichungsnummer : **0 380 501 B1**

(12) **EUROPÄISCHE PATENTSCHRIFT**

(45) Veröffentlichungstag der Patentschrift :
14.10.92 Patentblatt 92/42

(51) Int. Cl.$^5$ : **A61K 31/725**

(21) Anmeldenummer : 88907137.9

(22) Anmeldetag : 18.08.88

(86) Internationale Anmeldenummer :
PCT/CH88/00141

(87) Internationale Veröffentlichungsnummer :
WO 90/00058 11.01.90 Gazette 90/02

(54) GLYKOSAMINOGLYKAN ZUR BEHANDLUNG VON DIABETISCHER MIKROANGIOPATHIE.

Die Akte enthält technische Angaben, die nach dem Eingang der Anmeldung eingereicht wurden und die nicht in dieserPatentschrift enthalten sind.

(30) Priorität : 27.06.88 CH 2442/88

(43) Veröffentlichungstag der Anmeldung :
08.08.90 Patentblatt 90/32

(45) Bekanntmachung des Hinweises auf die Patenterteilung :
14.10.92 Patentblatt 92/42

(84) Benannte Vertragsstaaten :
AT BE CH DE FR GB IT LI LU NL SE

(56) Entgegenhaltungen :
Dialog Information Services, File 155, Medline 66-89, Nr. 02377939, M.G. Margolis et al.: "Atheroid and anginin in diabeticretinopathy", siehe Zusammenfassung, & Vestn Oftalmol (Ussr) 1973, 1 p. 38-41
Dialog Information Services, File 155, Medline 66-89, Nr. 04253053, L. Pampulov et al.: "Clinical and functional study of theevolution of diabetic macroangiopathy", siehe Zusammenfassung, & Vutr Boles 1980, 19 (5) p. 48-53
Dialog Information Services, File 155, Medline 66-89, Nr. 03697739, E.K. Bozadzhieva et al.: "Effect of heparin, heparinderivatives and heparinoids on blood sugar levels of diabetes mellitus patients", siehe Zusammenfassung, & Probl Endokrinol(Mosk) Nov-Dec 1978, 24 (6) p. 23-30

(73) Patentinhaber : REINER, Roland
Rheinfelderstrasse 8
CH-4058 Basel (CH)
Patentinhaber : REINER-STRUSCHKA, Helgard
Rheinfelderstrasse 8
CH-4058 Basel (CH)

(72) Erfinder : REINER, Roland
Rheinfelderstrasse 8
CH-4058 Basel (CH)
Erfinder : REINER-STRUSCHKA, Helgard
Rheinfelderstrasse 8
CH-4058 Basel (CH)

(74) Vertreter : Eder, Carl E.
Patentanwaltsbüro EDER AG
Lindenhofstrasse 40
CH-4052 Basel (CH)

(56) Entgegenhaltungen :

**Dialog Information Services, File 155, Medline 66-89, Nr. 03464451,S. Markoski et al.: "Effect of heparinoids on the bloodsugar level and blood lipids in diabetes mellitus", siehe Zusammenfassung, & Vutr Boles 1977, 16 (6) p. 52-8**

**The Merck Index. An Encyclopedia of Chemicals, Drugs, and Biologicals, 10. Auflage, 1983, Merck & Co., Inc. (Rahway, N.J,US), Seiten 314-315 siehe Seite 314, Nr. 2194, "Chondroitin sulfate"**

**The Merck Manual of Diagnosis and Therapy, 15. Auflage, 1987, Merck Sharp & Dohme Research Laboratories, (Rahway, N.J.,US), Seiten 386-389 siehe Seite 389**

**Rote Liste, 1976, 19016B, Eleparon**

**Interactions between Human Leucocyte Elastase and Chondroitin Sulfate", A.Baici et al., Chem. Biol. Interactions, Vol. 51,1984, Seiten 1-11.**

**"BIOCHEMIE"; L. Stryer, 3 Auflage, 1980, Seiten 156-158**

**"Römpps Chemie Lexikon", 8 Auflage , Band 13, Seiten 1674-1677**

**"Glycosaminoglycans from Ateroid.", Seethanathan et al., Molecular & Cellular Biochemistry, Vol. 8, 1975, Seiten 177-183**

**Arzneimittel-Verzeichnis der Roten Liste von 1991**

## Beschreibung

Die Erfindung bezieht sich auf die Herstellung eines Arzneimittels für die Therapie und Prophylaxe der diabetischen Mikroangiopathie.

Die diabetische Mikroangiopathie ist die wichtigste Spätkomplikation des Diabetes mellitus. Sie stellt eine generalisierte Krankheit des gesamten Kapillarsystems dar und äussert sich klinisch am gravierendsten in Form der diabetischen Retinopathie und der diabetischen Nephropathie (Glomerulosklerose); als kapillaropathisch-korrelierte Manifestationen kommen unter anderem die diabetische Parodontitis und die diabetische Hautgangrän hinzu. Die Mikroangiopathie ist die Hauptursache von Morbidität, Invalidität und Mortalität beim Diabetes. Die diabetische Retinopathie ist zudem die Hauptursache der Blindheit in den modernen Industrieländern. Die diabetische Mikroangiopathie ist durch erhöhte Permeabilität und Fragilität der Kapillargefässe gekennzeichnet. Dieser pathologische Zustand der Kapillaren ist auf ein deutliches Ungleichgewicht zwischen Biosynthese und Abbau des Kollagens der Basalmembran dieser Gefässe zurückzuführen. Typische Merkmale der so geschädigten Kapillaren sind die Verdickung der Basalmembran mit Verminderung des Proteoheparansulfat-Gehaltes und der selektive Untergang von intramuralen Perizyten. Dafür verantwortlich sind - mindestens teilweise - die nichtenzymatische Glykosylierung des Kollagens der Basalmembran, Autoimmunprozesse und die Protein-anabole Wirkung erhöhter Plasmakonzentrationen des Wachstumshormons und vor allem des Wachstumsfaktors IGF-I (insulin-like growth factor I), der identisch ist mit Somatomedin C bzw. dem "sulfation factor". Zu diesen Dysfunktionen gesellen sich oft weitere aggravierende Faktoren wie Hypertonie, Endothelschädigung der Kapillaren, Dyslipidämien, Hyperketonämie, Dysproteinämie, Plasmahyperviskosität, Thrombozyten-Hyperadhäsivität und -Hyperaggregation, Abnahme der Erythrozyten-Flexibilität, Zunahme der Erythrozyten-Aggregation, gesteigerte nichtenzymatische Glykosylierung des Hämoglobins unter Zunahme der Sauerstoffaffinität und geringerer Sauerstoffabgabe der Erythrozyten an die zu versorgenden Gewebe.

Dieser ganze Funktionsstörungskomplex kann, was das retinopathische Geschehen betrifft, zu Mikroaneurysmen, Exsudaten, Makulaödem, Mikrothromben, Kapillarokklusionen, Ischämie, Hypoxie, Netzhautblutungen, Gewebszerstörungen, hypoxisch-stimulierte und IGF-I- vermittelte Proliferationen neuer Gefässe in der Netzhautebene und in den Glaskörper hinein, Glaskörperblutungen und Netzhautablösung durch Traktion des schrumpfenden Glaskörpers, führen. Die Kontraktion bzw. Retraktion des Glaskörpers stellt eine typische, die diabetische Retinopathie begleitende Vitreopathie dar. Verantwortlich dafür ist eine Beeinträchtigung des stark wasserbindenden Kollagen-Hyaluronsäure-Netzwerkes des Glaskörpers durch das Aufkommen erhöhter Konzentrationen des Wachstumsfaktors IGF-I und von Abbauenzymen. Der Glaskörper verliert dabei seine ursprünglich gallertige Konsistenz und verflüssigt sich schliesslich.

Dass sich die diabetische Mikroangiopathie in der Netzhaut am frühesten und dramatischsten äussern kann, lässt sich zum Teil dadurch erklären, dass die Netzhaut das am meisten Sauerstoff verbrauchende Gewebe und die engsten Kapillaren des ganzen Organismus aufweist.

Es ist kein Medikament für eine wirkungsvolle Behandlung sowie Prophylaxe der diabetischen Mikroangiopathie und insbesondere für eine Verhinderung der durch die Mikroangiopathie verursachten und-/oder damit verknüpften Augenschäden bekannt. Solche Augenschäden werden beispielsweise dadurch behandelt, dass mit Laserstrahlen die Koagulation von Bereichen der Netzhaut bewirkt oder der Glaskörper operativ entfernt wird (Vitrektomie). Durch diese therapeutischen Massnahmen können jedoch eben höchstens gewisse durch die Mikroangiopathie verursachte Schädigungen der Augen gemildert und verzögert werden, während die Mikroangiopathie selbst und deren Auswirkungen auf die übrigen Körperbereiche nicht verhindert oder gar geheilt werden können.

Ferner sind an sich Glykosaminoglykane, darunter das Chondroitinsulfat, als antiarthrotische Wirkstoffe bekannt. Sie besitzen einerseits eine hemmende Wirkung auf die für die Gelenkknorpel-Degeneration verantwortlichen Enzyme und andererseits einen regulierenden Einfluss auf die biosynthetischen Leistungen der Chondrozyten, wobei auch die Ausgleichung des Defizits an Knorpelgrundsubstanz-Bausteinen von Bedeutung ist.

Der Erfindung liegt die Aufgabe zugrunde, die Herstellung eines Arzneimittels für die Therapie und Prophylaxe der diabetischen Mikroangiopathie zu ermöglichen.

Es wurde nun überraschend gefunden, dass Glykosaminoglykane, wie z. B. das Chondroitinsulfat, eine hohe Wirksamkeit als Therapeutika und Prophylaktika der diabetischen Mikroangiopathie besitzen.

Die gestellte Aufgabe wird daher gelöst durch die Verwendung eines Glykosaminoglykans, beispielsweise von Chondroitinsulfat oder von mindestens einem Chondroitinsulfat-Salz und/oder -Hydrat und/oder -Salzhydrat, wie z. B. Natrium-Chondroitinsulfat, zur Herstellung eines Arzneimittels für die Therapie und Prophylaxe der diabetischen Mikroangiopatie, wobei des Arzneimittel frei von Heparin, Heparansulfat, Dermatansulfat und Heparinoiden ist.

Die oben gennanten Glykosaminoglykane, wie z. B. das Chondroitinsulfat, d.h. ein saures, sulfatiertes Mucopolysaccharid, und dessen Salze und/oder Hydrate und/oder Salzhydrate, können zur Therapie und Prophylaxe der diabetischen Mikroangiopathie in Form von oralen, parenteralen und topischen Präparaten verwendet werden. Besonders vorteilhaft ist die orale Verabreichung von Chondroitinsulfat bzw. einem Salz von diesem. Die orale Tagesdosierung von Chondroitinsulfat bzw. dessen Salz ist zu Therapiebeginn 3 x 500 mg, die unter Umständen auf die Hälfte herabgesetzt oder verdoppelt werden kann. Diese Dosierung wird zwei bis vier Monate aufrechterhalten, bis sich eine Normalisierung der Kapillar-Permeabilität und -Resistenz eingestellt hat. Die Kontrolle erfolgt mittels Fluoreszenzangiographie der Netzhaut, Kapillarodynamometrie auf der Haut und Bindehaut sowie durch Bestimmung der Proteinurie. Nach erreichter Normalisierung der Kapillarfunktion folgt die Dauertherapie mit einer täglichen Erhaltungsdosierung von 3 x 250 mg Chondroitinsulfat, die sich unter Umständen verdoppeln lässt. Die Dauertherapie ist notwendig und entspricht dem chronischen Charakter der diabetischen Mikroangiopathie. Für die Prophylaxe der diabetischen Mikroangiopathie ist eine orale Tagesdosierung von ebenfalls 3 x 250 mg, unter Umständen von 3 x 500 mg Chondroitinsulfat angezeigt. Die Verträglichkeit von Chondroitinsulfat ist sehr gut.

Besonders wichtig ist es, mit der Therapie der diabetischen Mikroangiopathie so früh als möglich zu beginnen, d. h. bei den ersten ophthalmologischen Befunden (Ermittlung mittels Fluoreszenzangiographie der Netzhaut), bei beginnender Abnahme der Kapillarresistenz (Bestimmung mittels Kapillarodynamometrie auf der Haut und Bindehaut) und bei beginnender Proteinurie.

Eine allfällig vorhandene Hypertonie muss als ernster Aggravationsfaktor der diabetischen Mikroangiopathie betrachtet und mit einem für den Diabetiker gut verträglichen Antihypertonikum behandelt werden.

Als Präparat kann beispielsweise Natrium-Chondroitinsulfat verwendet werden, wie es als Wirkstoff in dem in der Schweiz unter der Handelsmarke "Structum" bekannten Medikament zur Behandlung von Arthrosen enthalten ist. Dieses Medikament ist in der Schweiz von der Firma Smith, Kline & French (Schweiz) AG, Luzern, bzw. der Vertriebsgesellschaft F. Uhlmann-Eyraud S.A., Genève, erhältlich.

Für die Herstellung eines Arzneimittels kann man 250 mg pulverförmiges Natrium-Chondroitinsulfat in eine Gelatine-Kapsel einfüllen und diese auf bekannte Art und Weise verschliessen. Solche Kapseln werden ganz geschluckt.

Eine andere Möglichkeit der Herstellung eines Arzneimittels besteht darin, dass man 50 g pulverförmiges Natrium-Chondroitinsulfat mit Saccharin-Natrium, einem Aromastoff, einem Farbstoff und Excipientien zu einem einheitlichen Gemisch verarbeitet und daraus 100 gleiche Portionen in Sachets abfüllt, die dann zum Einnehmen in einer Flüssigkeit aufgelöst werden.

## Patentansprüche

1. Verwendung mindestens eines Glykosaminoglykans zur Herstellung eines Arzneimittels für die Therapie und Prophylaxe der diabetischen Mikroangiopathie, wobei das Arzneimittel frei von Heparin, Heparansulfat, Dermatansulfat und Heparinoiden ist.

2. Verwendung nach Anspruch 1, wobei das Arzneimittel Chondroitin-4-sulfat und/oder Chondroitin-6-sulfat und/oder mindestens eines von deren Salzen und/oder Hydraten und/oder Salzhydraten enthält.

3. Verwendung nach Anspruch 2, wobei Natrium-Chondroitinsulfat zur Herstellung des Arzneimittels benutzt wird.

4. Verwendung nach einem der Ansprüche 1 bis 3, wobei das Arzneimittel zur oralen Verabreichung formuliert ist.

5. Verwendung nach einem der Ansprüche 1 bis 4, wobei das Arzneimittel eine der genannten Substanzen oder eine Mischung von solchen in einer Menge von mindestens 250 mg pro Dosiseinheit enthält.

## Claims

1. Use of at least one glycosaminoglycan for the production of a medicine for the therapy and prophylaxis of diabetic microangiopathy, wherein the medicine is free of heparin, heparan sulfate, dermatan sulfate and heparinoids.

4

2. Use according to claim 1, wherein the medicine contains chondroitin-4-sulfate and/or chondroitin-6-sulfate and/or at least one of the salts and/or hydrates and/or salt hydrates thereof.

3. Use according to claim 2, wherein sodium chondroitin sulfate is used for production of the medicine.

4. Use according to one of claims 1 to 3, wherein the medicine is formulated for oral administration.

5. Use according to one of claims 1 to 4, wherein the medicine contains one of the said substances or a mixture of such in a quantity of at least 250 milligrams per dosage unit.

**Revendications**

1. Utilisation d'au moins un glycosaminoglycan pour la fabrication d'un médicament pour le traitement et la prophylaxie de la microangiopathie diabétique, ledit médicament étant exempt d'héparine, de sulfate d'héparane, de sulfate de dermatane et d'héparinoïdes.

2. Utilisation selon la revendication 1, ledit médicament contenant du sulfate de 4-chondroïtine et/ou du sulfate de 6-chondroïtine et/ou au moins l'un de leurs sels et/ou hydrates et/ou sels hydrates.

3. Utilisation selon la revendication 2, du sulfate de chondroïtine de sodium étant utilisé pour la fabrication du médicament.

4. Utilisation selon l'une des revendications 1 à 3, le médicament étant formulé pour l'administration orale.

5. Utilisation selon l'une des revendications 1 à 4, le médicament contenant l'une des substances mentionnées ou un mélange de ces substances en une quantité d'au moins 250 mg par dose unitaire.